(19) Europäisches Patentamt
European Patent Office
Office européen des brevets

(11) Publication number: **0 038 298**
**B1**

(12) **EUROPEAN PATENT SPECIFICATION**

(45) Date of publication of patent specification: **25.07.84**

(21) Application number: **81810131.3**

(22) Date of filing: **01.04.81**

(51) Int. Cl.³: **C 07 D 413/04,**
C 07 D 261/08,
A 61 K 31/42 //(C07D413/04,
209/14, 261/08)

# (54) Isoxazolyl indolamines.

(30) Priority: **10.04.80 US 138872**
**14.10.80 US 196785**
**10.04.80 US 138873**

(43) Date of publication of application:
**21.10.81 Bulletin 81/42**

(45) Publication of the grant of the patent:
**25.07.84 Bulletin 84/30**

(84) Designated Contracting States:
**AT BE CH DE FR GB IT LI LU NL SE**

(56) References cited:
**GB - A - 1 075 156**
**US - A - 4 059 583**

(73) Proprietor: **SANDOZ AG**
**Lichtstrasse 35**
**CH-4002 Basel (CH)**

(72) Inventor: **Brand, Leonard Jay**
**114 Dover Chester Road**
**Randolph N.J. 07801 (US)**
Inventor: **Nadelson, Jeffrey**
**12 Benedict Crescent**
**Denville N.J. 07834 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 038 298 B1

## Description

This application relates to novel isoxazolyl indolamines, their acid addition and alkali metal salts, processes for their production and their use as pharmaceuticals in particular anti-diabetic agents.

In particular the invention relates to compounds of formula I

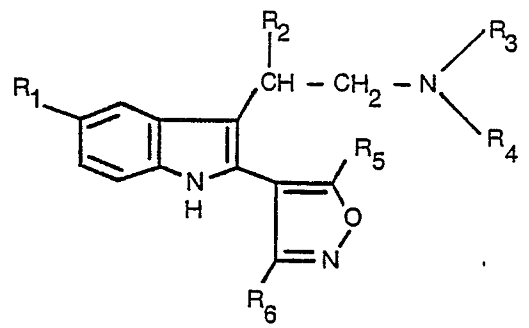

I

wherein

$R_1$ represents hydrogen, fluorine, chlorine, $C_1$—$C_4$alkyl or $C_1$—$C_4$alkoxy,

$R_2$ represents hydrogen or hydroxyl,

$R_3$ and $R_4$ represent, independently, $C_1$—$C_4$alkyl or together with the adjacent nitrogen atom

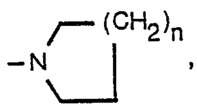 , 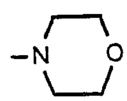 or 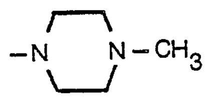

wherein

n is 1, 2 or 3,

$R_5$ represents hydrogen or $C_1$—$C_4$alkyl and

$R_6$ represents hydrogen, $C_1$—$C_4$alkyl, phenyl or phenyl mono-substituted by fluorine, chlorine, $C_1$—$C_4$alkyl or $C_1$—$C_4$alkoxy.

The present invention further provides a process for the production of compounds of formula I which comprises reducing the corresponding compound of formula II

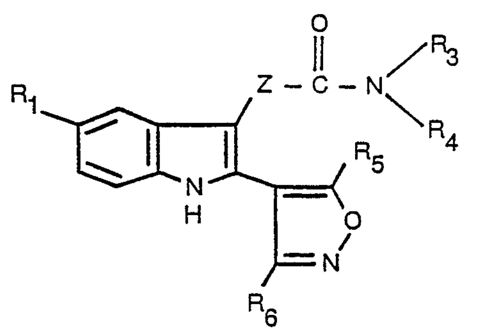

II

wherein

$R_1$, $R_3$, $R_4$, $R_5$ and $R_6$ are as defined above and

Z signifies —CO— or —$CH_2$—.

The reduction according to the process is suitably carried out under an inert atmosphere of, for example, helium, argon or preferably nitrogen and in an inert solvent such as an ether e.g. diethylether, dioxane or tetrahydrofuran.

Examples of reducing catalysts which may be used when carrying out the process are those common for the reduction of carbonyl groups e.g. diborane and alkali metal hydride reducing agents e.g. sodium borohydride or, preferably, lithium aluminium hydride.

It will be appreciated that when Z is —CO, depending on the conditions employed, reduction will be either partial (to give compounds of formula I wherein $R_2$ represents hydroxyl) or complete (to give compounds of formula I wherein $R_2$ represents hydrogen) and conditions will thus be chosen according to the desired final product.

In cases where a final product is desired in which $R_2$ represents hydroxyl, temperatures may thus be critical and a preferred range is from 10°C to 30°C, most preferably from 20°C to 25°C. If complete

reduction to final products wherein $R_2$ represents hydrogen is desired, a temperature range of from 60°C to 150°C is preferred but is not critical.

It will be appreciated that partial reduction can also be achieved by selection of a suitably weak reducing agent.

Alternatively, compounds of formula I in which $R_2$ is H may be produced using compounds of formula II in which Z is —$CH_2$—. The preferred temperatures in this case lie between 0° to 50°C, most preferably between 20° and 30°C. This embodiment represents the preferred method of producing compounds in which $R_6$ is $C_{1-4}$alkyl.

The compounds of formula II can be prepared by reacting a compound of formula III

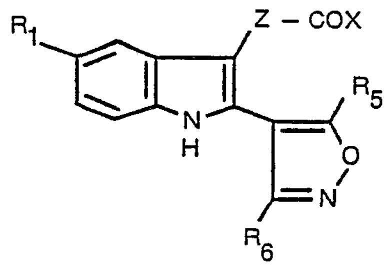

III

with a compound of formula IV

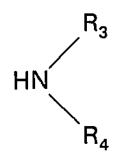

IV

wherein

X represents a leaving group such as chlorine or bromine and

$R_1$, $R_3$, $R_4$, $R_5$ and $R_6$ are as defined above.

The reaction is carried out in the presence of an inert solvent. Examples of preferred solvents include water, an excess of a compound of the formula IV or an ether such as diethylether, dioxane or tetrahydrofuran, or an aromatic hydrocarbon such as benzene or toluene preferably the combination of water, diethylether or tetrahydrofuran and excess compound of formula IV. The preferred temperature is from −10° to 50°C, most preferably from 20° to 30°C.

The compounds of formula III in which Z is —CO— can be prepared by reacting a compound of formula V

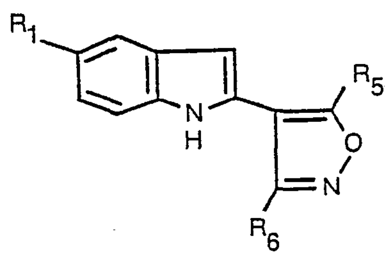

V

with a compound of formula VI

$$(COX)_2 \qquad VI$$

wherein $R_1$, $R_5$, $R_6$ and X are as defined above.

The reaction is carried out in the presence of an inert solvent. Examples of preferred solvents include an ether such as diethylether, dioxane or tetrahydrofuran or an aromatic hydrocarbon such as benzene or toluene, preferably diethylether.

The preferred temperature is from −10° to 50°C, most preferably from 20° to 30°C.

The compounds of formula V can be prepared by cyclising a compound of formula VII

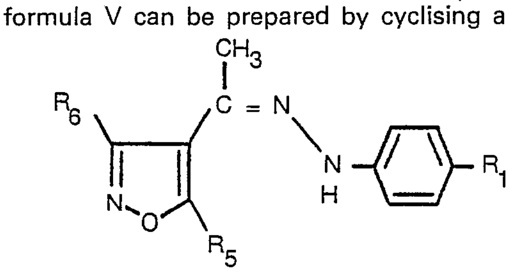

VII

wherein $R_1$, $R_5$ and $R_6$ are as defined above.

The cyclisation is preferably carried out in the presence of an acid such as acetic acid, p-toluenesulphonic acid or, preferably, polyphosphoric acid, and in the presence of an inert solvent such as an aromatic hydrocarbon e.g. benzene or toluene or preferably an excess of said acid. Preferred temperatures are of from 70° to 150°C, most preferably 105—120°C.

The compounds of formula VII can be prepared by reacting a compound of formula VIII

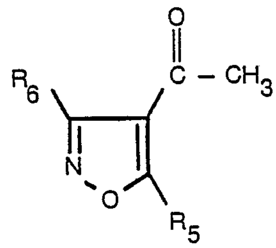

VIII

with a compound of formula IX

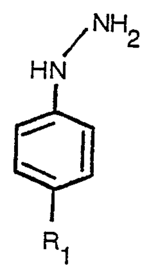

IX

wherein $R_1$, $R_5$ and $R_6$ are as defined above.

The reaction is preferably carried out in the presence of an acid such as sulphuric acid, a polyphosphoric acid or preferably *p*-toluene sulphonic acid and in the presence of an inert solvent. Examples of preferred solvents are aromatic hydrocarbons such as benzene or toluene or a lower alkanol such as methanol and most preferably ethanol. Preferred temperatures are of from 0°—100°C, most preferably 20° to 35°C.

The compounds of formula III in which Z is $—CH_2—$ can be prepared by introducing the X group into a compound of formula Xa.

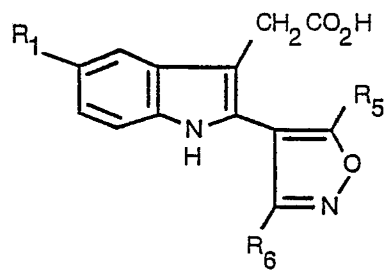

Xa

wherein $R_1$, $R_5$ and $R_6$ are as defined above.

When X is halogen, for example a suitable agent for its introduction is e.g. thionyl chloride or bromide or phosphorous oxychloride. The remaining reaction conditions are similar to those described for the reaction of compounds of formula V and formula VI.

The compounds of formula Xa can be prepared by reacting a compound of formula XI

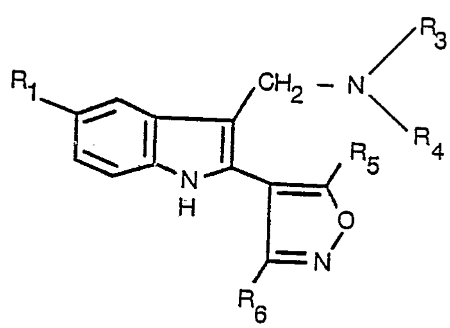

XI

wherein $R_1$ and $R_3$ to $R_6$ are as defined above.

with an alkali metal cyanide such as sodium or potassium cyanide.

The reaction is effected in the presence of an aqueous alcoholic solvent. Suitable alcohols are e.g. lower alkanols such as methanol and preferably ethanol. Preferred reaction temperatures are from 80° to 150°C.

This reaction leads to a mixture of the compound of formula Xa and the corresponding amide of formula Xb

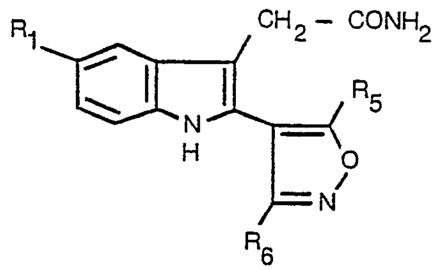

Xb

which can be converted to the compound of formula Xa by conventional hydrolysis techniques.

The compounds of formula XI can be prepared by reacting a compound of formula V with a compound of formula IV both as defined above in the presence of formaldehyde, an acid catalyst such as acetic acid and an organic co-solvent. Examples of suitable solvents are ethers such as diethylether, tetrahydrofuran and particularly dioxane. Preferred temperatures are from about −10° to +30°C, most preferably 0° to 10°C.

The products resulting from the above processes can be isolated and purified in conventional manner. Intermediate compounds can where appropriate be further reacted without isolation.

Insofar as the production of other starting materials is not described above, these are either known or can be prepared in conventional manner from known starting materials.

Depending on the conditions employed the compounds of formula I may be obtained either in free base form or in acid addition salt form. The acid addition salt forms of the compounds of formula I also form part of the invention.

Free base forms of formula I may be converted into acid addition salt forms in conventional manner and vice versa.

Examples of suitable salt forming acids are hydrochloric, hydrobromic, hydroiodic, phosphorous, sulphuric, acetic, maleic and fumaric.

The compounds of formula I may also be isolated in the form of salts with bases, e.g. alkali metal salts such as sodium or potassium salts. Such salt forms also form part of the invention.

It will be understood that compounds of formula I wherein $R_2 = OH$ may exist in racemic form or in the form of optically active isomers which can be produced or separated and isolated from the racemate in conventional manner. The invention extends to such isomeric forms as well as racemic forms.

The compounds of the formula I possess pharmacological acticity. In particular, they possess hypoglycemic activity as indicated by the lowering of blood glucose in 6 to 8 week old male Royal Hart mice weighing 30 to 35 gram which are fasted in groups of 5 for 16 hours and then are given an initial dose of 50 to 200 milligrams per kilogram of animal body weight of the compound orally. Two hours after the test compound is administered the mice are anesthetized with 85 milligrams per kilogram of animal body weight of sodium hexobarbital and five minutes later blood is collected via cardiac puncture. The blood samples are placed in an autoanalyser cup containing 0.025 millilitres of heparin (1.000 units per millilitres); and the samples are capped, shaken and stored in ice. The glucose level is determined by the autoanalyser potassium ferric cyanide N-2b method and these glucose levels are then compared with the glucose levels of the control group which receives orally 0.5% carboxymethyl cellulose and is run concurrently. To validate this experiment, a known hypoglycemic standard is included each time the test is run. The compounds are thus indicated for use as in the treatment of diabetes as hypoglycemic agents.

The compounds of formula I also inhibit or impede post-prandial hyperglycemia as indicated by a lowering of the blood sugar levels in male Wistar rats after an oral starch load. In this test male Wistar rats in groups of 5 which are fasted for 16 hours are given an intial dose of from 25 to 200 mg/kg p.o. of the test compound. One hour later the rats are given 1.0 grams per kilogram of animal body weight of cooked starch load. Thirty minutes after administration of the starch, the rats are anesthetized with 120 milligrams per kilogram of animal body weight of sodium hexobarbital after which blood is collected via cardiac puncture. The blood samples are placed in an autoanalyser cup containing 0.1 millilitres of heparin (1.000 units per millilitres). The heparinized blood is used to determine the blood sugar level with an autoanalyser. The blood sugar content is compared to the control group which receives 0.5% carboxymethyl cellulose and an oral starch load and are run concurrently.

The compounds are thus also indicated for use in the treatment of diabetes by inhibiting or impeding post-prandial hyperglycemia.

An indicated suitable daily dosage in the treatment of diabetes as hypoglycemic agents is from 50 to 2000 mg suitably administered in divided dose of 12.5 to 1000 mg, two to four times daily or in retard form.

An indicated suitable daily dosage for inhibiting or impeding post-prandial hyperglycemia in the treatment of diabetes is from 25 to 1000 mg preferably administered at mealtimes e.g. three times a day in divided dosages of from about 10 to 400 mg.

The invention therefore also concerns compounds of formula I for use as pharmaceuticals e.g. as hypoglycemic agents or as agents for inhibiting or impeding post-prandial hyperglycemia.

The compounds of formula I may be administered in free base form or in the form of pharmaceutically acceptable acid addition or alkali metal salts, which salt forms have the same order of activity as the free forms.

The compounds of formula I or their pharmaceutically acceptable acid addition or alkali metal salts may be administered alone, or in admixture with a pharmaceutically acceptable diluent or carrier, and, optionally other excipients, and administered orally in such forms as tablets, elixirs, capsules or suspensions or parenterally in such forms as injectable solutions or suspensions.

The preferred pharmaceutical compositions from the stand-point of ease of preparation and administration are solid compositions, particularly tablets and hard-filled or liquid-filled capsules.

Such compositions also form part of the invention.

The following examples, in which all temperatures are in °C and room temperatures is 20—30°C, illustrate the invention.

Example 1

1-(3-Ethyl-5-methyl-4-isoxazolyl)-1-ethanone phenyl hydrazone (Compound of formula VII)

A mixture of 61.1 g (0.4 mol) of 4-acetyl-3-ethyl-5-methyl-isoxazole, 39.4 ml (0.4 mol) of phenyl hydrazine and 500 mg toluenesulphonic acid in 400 ml ethanol is stirred at room temperature for 48 hours. The resulting solid is filtered and washed with cold ether to give 1-(3-ethyl-5-methyl-4-isoxazolyl)-1-ethanone phenyl hydrazone; m.p. 72° to 75°.

Example 2

2-(3-Ethyl-5-methyl-4-isoxazolyl)-indole (Compound of formula V)

To 1350 grams of polyphosphoric acid at 100° to 110° there is added portionwise 74.5 g (0.307 mol) of 1-(3-ethyl-5-methyl-4-isoxazolyl)-1-ethanone phenyl hydrazone while maintaining the temperature between 105° and 115°. After addition is complete, the mixture is stirred at 100° to 110° for 3 hours. The mixture is then poured onto ice and water and the resulting gum extracted into methylene chloride. The methylene chloride is decolorized, dried over anhydrous magnesium sulphate, filtered and evaporated in vacuo to give 2-(3-ethyl-5-methyl-4-isoxazolyl)-indole.

Example 3

2-(3-Ethyl-5-methyl-4-isoxazolyl)-3-indole-glyoxyl chloride (Compound of formula III)

A mixture of 163 g (0.72 mol) of 2-(3-ethyl-5-methyl-4-isoxazolyl)-indole and 3 litres of ether are cooled to 0° to 10° and 61.6 ml (0.72 mol) of oxalyl chloride in 450 ml of ether is added dropwise, maintaining the temperature at 0° to 10° during the addition. The cooling bath is removed and the mixture allowed to warm to room temperature. After $1\frac{1}{2}$ hours, some starting material is still present and 3.1 ml (0.0362 mol) of oxalyl chloride is added and the mixture stirred for an additional $1\frac{1}{2}$ hours. Total stirring at room temperature is 3 hours. The solvent is evaporated in vacuo to give a solid residue. The residue is suspended in ether and evaporated twice more to give 2-(3-ethyl-5-methyl-4-isoxazolyl)-3-indole-glyoxyl chloride.

Example 4

N,N-dimethyl-2-(3-ethyl-5-methyl-4-isoxazolyl)-3-indole-glyoxyl amide (Compound of formula II)

79.0 g (0.250 mol) of 2-(3-ethyl-5-methyl-4-isoxazolyl)-3-indole-glyoxyl chloride is added portion-wise to a 0° to 10° cooled mixture of 1200 ml of 40% aqueous dimethylamine and 1000 ml of ether. The resulting mixture is stirred for 1 hour without an ice bath and then filtered. The solid is then washed well with water and with three portions of cold ether to give N,N-dimethyl-2-(3-ethyl-5-methyl-4-isoxazolyl)-3-indoleglyoxylamide; m.p. 212° to 215°.

The following compounds of formulae II, III, V and VII may be prepared analogously to Examples 1 to 4 or by any other method as hereinbefore described.

TABLE I ($Z = \overset{O}{\overset{\|}{C}}$)

(* = cmpds. of Examples 1 to 4)

| Cmpd. No. | $R_1$ | $R_5$ | $R_6$ | V | VII | $R_3$ | $R_4$ | II |
|---|---|---|---|---|---|---|---|---|
| a* | H | $CH_3$ | $C_2H_5$ | | m.p. 72–75° | $CH_3$ | $CH_3$ | m.p. 221–223° |
| b | | H | $C_2H_5$ | | | | | m.p. 211–212° |
| c | | $C_2H_5$ | $C_2H_5$ | | | | | m.p. 218–219° |
| d | | $i$-$C_3H_7$ | $C_2H_5$ | | | | | |
| e | | $t$-$C_4H_9$ | $C_2H_5$ | | | | | |
| f | | $CH_3$ | $CH_3$ | | | | | m.p. 211–214° |
| g | | $CH_3$ | H | | | | | |
| h | | $CH_3$ | $n$-$C_3H_7$ | | | | | m.p. 153–154° |
| i | | $CH_3$ | $t$-$C_4H_9$ | | | | | m.p. 270° (dec.) |
| j | | $C_2H_5$ | $CH_3$ | | | | | m.p. 155–156° |
| k | | $CH_3$ | $i$-$C_3H_7$ | | | | | m.p. 231–233° |
| l | F | | $C_2H_5$ | | | | | |
| m | $CH_3$ | | | | | | | |
| n | $OCH_3$ | | | | | | | |
| o | H | | | | | $C_2H_5$ | $C_2H_5$ | m.p. 2–1–213° |
| p | H | | | | | 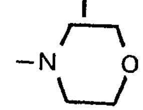 | | |

TABLE I (Continued)

| Cmpd. No. | $R_1$ | $R_5$ | $R_6$ | V | VII | $R_3$ | $R_4$ | II |
|---|---|---|---|---|---|---|---|---|
| q | H | $CH_3$ | $C_2H_5$ | | | 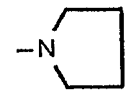 | | m.p. 194–197° |
| r | H | | $C_2H_5$ | | | 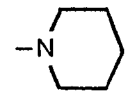 | | m.p. 199–203° |
| s | F | | $C_2H_5$ | | | 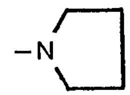 | | |
| t | H | | phenyl | m.p. 145–146° | m.p. 129–133° | $CH_3$ | $CH_3$ | m.p. 259–261° |
| u | F | | | | | $CH_3$ | $CH_3$ | |
| v | $CH_3$ | | | | | $CH_3$ | $CH_3$ | |
| w | $OCH_3$ | | | | | $CH_3$ | $CH_3$ | |
| x | H | | | | | 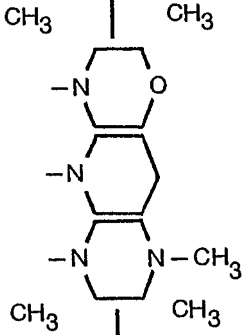 | | m.p. 210–221° |
| y | H | | | | | | | m.p. 193–195° |
| z | H | | | | | | | |
| aa | F | | $CH_3$ | | | $CH_3$ | $CH_3$ | m.p. 220–222° |
| ab | F | $C_2H_5$ | $CH_3$ | | | $CH_3$ | $CH_3$ | |
| ac | F | $CH_3$ | $CH_3$ | | | 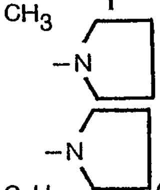 | | |
| ad | F | $CH_3$ | $CH_3$ | | | | | |
| ae | H | $CH_3$ | phenyl | | | $C_2H_5$ | $C_2H_5$ | |

Example 5

α-(Dimethylaminomethyl)-2-(3-ethyl-5-methyl-4-isoxazolyl)-1H-indole-3-methanol (Compound of formula I in which $R_2$ represents hydroxyl)

To a suspension of 9.9 g (0.26 mol) of lithium aluminium hydride and 950 ml tetrahydrofuran under nitrogen there is added 21.0 g (0.065 mol) of N,N-dimethyl-2-(3-ethyl-5-methyl-4-isoxazolyl)-3-indoleglyoxylamide in 735 ml of tetrahydrofuran, while maintaining the temperature between 20° to 25°. After addition is complete, the mixture is stirred at room temperature for 5 hours then cooled to 0° and quenched by the addition dropwise of 20 ml of water in 180 ml of tetrahydrofuran. The resulting solids are filtered and the tetrahydrofuran dried over anhydrous magnesium sulphate, filtered and evaporated. The residue is crystallized from ethanol to give α-(dimethylaminomethyl)-2-(3-ethyl-5-methyl-4-isoxazolyl)-1H-indole-3-methanol; m.p. 178° to 180°.

Example 6

2-(3-ethyl-5-methyl-4-isoxazolyl)-3-(dimethylaminoethyl)-indole (Compound of formula I in which $R_2$ represents hydrogen)

To a refluxing suspension of 22.8 g (0.60 mol) of lithium aluminium hydride in 1100 ml of tetrahydrofuran under nitrogen, there is added dropwise over approximately 1 to 1½ hours a warm solution of 48.7 g (0.15 mol) of N,N-dimethyl-2-(3-ethyl-5-methyl-4-isoxazolyl)-3-indole-glyoxyl amide in 2250 ml of tetrahydrofuran. The mixture is refluxed for 3 hours after addition is completed. The resulting suspension is cooled to −10° to +5° and a solution of 270 ml of tetrahydrofuran and 90 ml of water is added dropwise with the temperature maintained between −10° and +5°. The suspension is stirred for approximately 1 hour without a cooling bath and then allowed to stand overnight without stirring. The mixture is filtered, the filtrate evaporated in vacuo and the residue is dissolved in methylene chloride. The methylene chloride is washed with water, dried and evaporated in vacuo. The residue is crystallized from ether to give 2-(3-ethyl-5-methyl-4-isoxazolyl)-3-dimethylaminoethyl)-indole; m.p. 118° to 120°.

Example 7

2-(3-Ethyl-5-methyl-4-isoxazolyl)-3-(dimethylaminoethyl)-indole (Compound of formula I wherein $R_2$ is hydrogen

*Step A) 2-(3-Ethyl-5-methyl-4-isoxazolyl)-3-(dimethylaminomethyl)-indole (Compound of formula XI)*

A mixture of 20.6 ml (0.24 mol) 37% aqueous formaldehyde, 18 ml (0.12 mol) 40% aqueous dimethylamine and 80 ml acetic acid is cooled to 0° and treated by the dropwise addition of 25.5 g (0.113 mol) 2-(3-ethyl-5-methyl-4-isoxazolyl)-indole in a solution of 45 ml acetic acid and 125 ml dioxane. After addition is complete the mixture is stirred for 1 hour at room temperature and poured onto 500 ml ice-water. The resulting solution is made basic with 20% potassium hydroxide and then extracted with methylene chloride. The methylene chloride is washed with water, brine, dried over anhydrous magnesium sulphate, filtered and evaporated to give a gum that crystallizes to give 2-(3-ethyl-5-methyl-4-isoxazolyl)-3-(dimethylaminomethyl)-indole; m.p. 88° to 90°.

*Step B) 2-(3-Ethyl-5-methyl-4-isoxazolyl)-3-indole acetic acid (Compound of formula Xa)*

A mixture of 24.8 g (0.088 mol) of 2-(3-ethyl-5-methyl-4-isoxazolyl)-3-(dimethylaminomethyl)-indole and 23 g (0.47 mol) of sodium cyanide in 175 ml ethanol and 50 ml water are refluxed for 80 hours. The mixture is then cooled and poured onto water, stirred for ½ hour and filtered to yield 2-(3-ethyl-5-methyl-4-isoxazolyl)-3-indole acetamide; m.p. 194° to 195°. The filtrate, which contains the sodium salt of 2-(3-ethyl-5-methyl-4-isoxazolyl)-3-indole acetic acid is evaporated to remove ethanol and acidified with cold 12N hydrochloric acid and the precipitate filtered to give 2-(3-ethyl-5-methyl-4-isoxazolyl)-3-indole acetic acid; m.p. 194° to 197°.

*Step C) 2-(3-Ethyl-5-methyl-4-isoxazolyl)-3-indole acetyl chloride (Compound of formula III)*

A mixture of 10.3 g (0.036 mol) 2-(3-ethyl-5-methyl-4-isoxazolyl)-3-indole acetic acid and 22.0 g (0.185 mol) thionyl chloride in 100 ml toluene is stirred at room temperature for 18 hours. The toluene is removed in vacuo to give 2-(3-ethyl-5-methyl-4-isoxazolyl)-3-indole acetyl chloride.

*Step D) N,N-dimethyl-2-(3-ethyl-5-methyl-4-isoxazolyl)-3-indoleacetamide (Compound of formula II)*

A mixture of 11.2 g (0.036 mol) of 2-(3-ethyl-5-methyl-4-isoxazolyl)-3-indole acetyl chloride in 100 ml of tetrahydrofuran is added to a vigorously stirred solution of dimethylamine in water (25 ml 40% dimethylamine in water) at 0° to 10°. The resulting mixture is allowed to warm to room temperature and poured into ether-water. The organic layer is separated, dried over anhydrous magnesium sulphate, filtered and evaporated to give N,N-dimethyl-2-(3-ethyl-5-methyl-4-isoxazolyl)-3-indole acetamide; m.p. 176° to 178°.

*Step E) 2-(3-ethyl-5-methyl-4-isoxazolyl)-3-dimethylaminoethyl)-indole*

A solution of 4.7 g (0.015 mol) of N,N-dimethyl-2-(3-ethyl-5-methyl-4-isoxazolyl)-3-indoleacetamide in 47 ml of tetrahydrofuran under nitrogen is added dropwise to a suspension of 2.28 g (0.06

mol) lithium aluminium hydride in 50 ml tetrahydrofuran, while maintaining the reaction at room temperature. The mixture is stirred for 1½ hours at room temperature, cooled in ice and quenched with 5 ml water in 50 ml tetrahydrofuran. The resulting solids are filtered and the filtrate dried over anhydrous magnesium sulphate, filtered and evaporated and the residue crystallized from ether to give 2-(3-ethyl-5-methyl)-4-isoxazolyl)-3-(dimethylaminoethyl)-indole; m.p. 118° to 120°.

The following compounds of formula I can be prepared analogously to the appropriate Examples 5 to 7 or according to any other method as hereinbefore described.

TABLE 2

| Cmpd. No. | $R_1$ | $R_2$ | $R_3$ | $R_4$ | $R_5$ | $R_6$ | Physical data |
|---|---|---|---|---|---|---|---|
| 1 (Ex. 5) | H | OH | $CH_3$ | $CH_3$ | $CH_3$ | $C_2H_5$ | m.p. 178–180° |
| 2 | | | | | H | $C_2H_5$ | m.p. 148–150° |
| 3 | | | | | $C_2H_5$ | $C_2H_5$ | m.p. 148–149° |
| 4 | | | | | $i$-$C_3H_7$ | $C_2H_5$ | |
| 5 | | | | | $t$-$C_4H_9$ | $C_2H_5$ | |
| 6 | | | | | $CH_3$ | $CH_3$ | m.p. 188–189° |
| 7 | | | | | $CH_3$ | H | |
| 8 | | | | | $CH_3$ | $n$-$C_3H_7$ | m.p. 176–178° |
| 9 | | | | | $CH_3$ | $t$-$C_4H_9$ | m.p. 205–206° |
| 10 | | | | | $C_2H_5$ | $CH_3$ | m.p. 153–155° |
| 11 | | | | | $CH_3$ | $i$-$C_3H_7$ | m.p. 186–188° |
| 12 | F | | | | | $C_2H_5$ | m.p. 125–127° |
| 13 | $CH_3$ | | | | | | |
| 14 | $OCH_3$ | | | | | | |
| 15 | H | | $C_2H_5$ | $C_2H_5$ | | | m.p. 153–154° |
| 16 | H | | 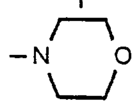 | | | | m.p. 169–171° |
| 17 | H | | 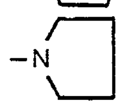 | | | | m.p. 166–168° |
| 18 | H | | 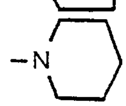 | | | | m.p. 166–167° |
| 19 | F | | 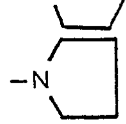 | | | | m.p. 159–160° |
| 20 | H | H | $CH_3$ | $CH_3$ | | | m.p. 118–120° |
| 21 | | | | | H | | |
| 22 | | | | | $C_2H_5$ | | |
| 23 | | | | | $i$-$C_3H_7$ | | |

TABLE 2 (Continued)

| Cmpd. No. | $R_1$ | $R_2$ | $R_3$ | $R_4$ | $R_5$ | $R_6$ | Physical data |
|---|---|---|---|---|---|---|---|
| 24 | H | H | $CH_3$ | $CH_3$ | $t$-$C_4H_9$ | $C_2H_5$ | |
| 25 | | | | | $CH_3$ | $CH_3$ | |
| 26 | | | | | | H | |
| 27 | | | | | | $n$-$C_3H_7$ | |
| 28 | | | | | | $t$-$C_4H_9$ | |
| 29 | F | | | | | $C_2H_5$ | |
| 30 | $CH_3$ | | | | | | |
| 31 | $OCH_3$ | | | | | | |
| 32 | H | | $C_2H_5$ | $C_2H_5$ | | | |
| 33 | | | 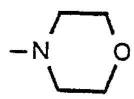 | | | | |
| 34 | | | 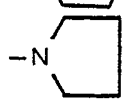 | | | | |
| 35 | | | 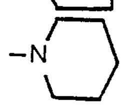 | | | | |
| 36 | | | $CH_3$ | $CH_3$ | | phenyl | m.p. 141–143.5° |
| 37 | F | | | | | | m.p. 152–154° |
| 38 | $CH_3$ | | | | | | |
| 39 | $OCH_3$ | | | | | | |
| 40 | H | | 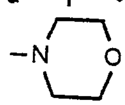 | | | | m.p. 104–105° |
| 41 | H | | 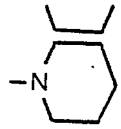 | | | | m.p. 150–151° |
| 42 | H | | 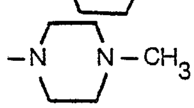 | | | | |
| 43 | F | OH | $CH_3$ | $CH_3$ | | $CH_3$ | m.p. 175–178° |
| 44 | F | OH | $CH_3$ | $CH_3$ | $C_2H_5$ | $CH_3$ | m.p. 79–80° |

TABLE 2 (Continued)

| Cmpd. No. | $R_1$ | $R_2$ | $R_3$ | $R_4$ | $R_5$ | $R_6$ | Physical data |
|---|---|---|---|---|---|---|---|
| 45 | F | OH | 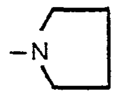 | | $CH_3$ | $C_2H_5$ | m.p. 168–169° |
| 46 | F | OH | 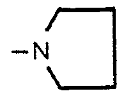 | | $CH_3$ | $CH_3$ | |
| 47 | H | H | $C_2H_5$ | $C_2H_5$ | $CH_3$ | phenyl | m.p. 110–112° |

Examples of preferred compound groups are for example those wherein

a) $R_3$ and $R_4$ represent, independently, $C_1$—$C_4$alkyl or together with the adjacent nitrogen atom

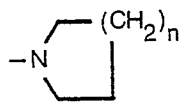 or 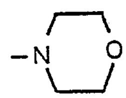 ,

$R_5$ and $R_6$ represent, independently, hydrogen or $C_1$—$C_4$alkyl and $R_1$, $R_2$ and n are as defined above.

Within this group compounds wherein $R_2$ represents hydroxyl are especially preferred.

b) $R_2$ represents hydrogen

$R_3$ and $R_4$, independently, represent $C_1$—$C_4$alkyl or together with the adjacent nitrogen atom

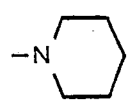 , 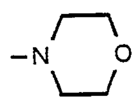 or 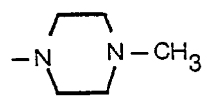

$R_5$ represents methyl,

$R_6$ represents phenyl or phenyl mono-substituted by fluorine, chlorine, $C_{1-4}$alkyl or $C_{1-4}$alkoxy and $R_1$ is as defined above.

Within these groups and generally for compounds of formula I the following meanings for the individual substituents or combinations thereof are preferred.

$R_1$ = a) H, F, $CH_3$, $OCH_3$;

b) H

$R_3 = R_4 = CH_3$;

$R_3 + R_4 + N =$ 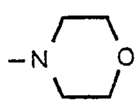 , 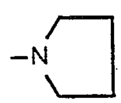 , 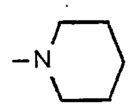 ;

$R_5$ and $R_6$, independently,

a) H, $C_{1-4}$alkyl

b) $C_{1-2}$alkyl or

c) $R_5 = CH_3$ $R_6$ = phenyl

d) $R_5 = CH_3$ $R_6 = C_{1-4}$alkyl

e) $R_5 = C_{1-4}$alkyl $R_6 = C_2H_5$

f) $R_5 = CH_3$ $R_6 = C_2H_5$

A particularly preferred compound is α-(dimethylaminomethyl)-2-(3-ethyl-5-methyl-4-isoxazolyl)-1H-indole-3-methanol.

**Claims for the Contracting States: BE CH DE FR GB IT LI LU NL SE**

1. Compounds of formula I

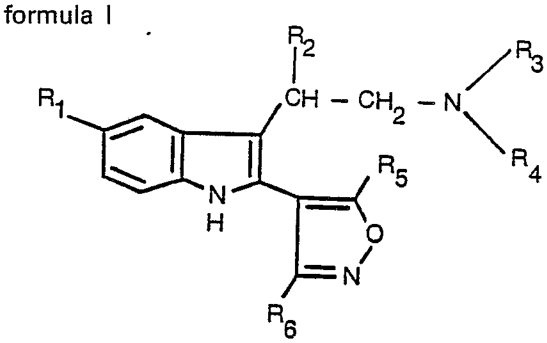

I

wherein

$R_1$ represents hydrogen, fluorine, chlorine, $C_{1-4}$alkyl or $C_{1-4}$alkoxy,

$R_2$ represents hydrogen or hydroxyl,

$R_3$ and $R_4$ represent, independently, $C_{1-4}$alkyl or together with the adjacent nitrogen atom

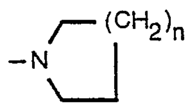 , 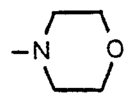 or 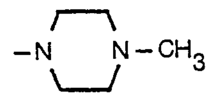

wherein

n is 1, 2 or 3,

$R_5$ represents hydrogen or $C_{1-4}$alkyl and

$R_6$ represents hydrogen, $C_{1-4}$alkyl, phenyl or phenyl mono-substituted by fluorine, chlorine, $C_{1-4}$alkyl or $C_{1-4}$alkoxy.

2. A compound as Claimed in Claim 1 wherein

a) $R_3$ and $R_4$ represent independently $C_{1-4}$alkyl or together with the adjacent nitrogen atom

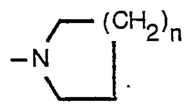 or 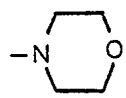 ,

and

$R_5$ and $R_6$ represent independently hydrogen or $C_{1-4}$alkyl,

b) $R_3$ and $R_4$ represent independently $C_{1-4}$alkyl or together with the adjacent nitrogen atom

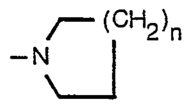 or 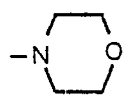 ,

and

$R_2$ represents hydroxyl or

c) $R_2$ represent hydrogen, $R_3$ and $R_4$ independently represent $C_{1-4}$alkyl or together with the adjacent nitrogen atom

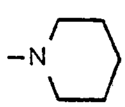 , 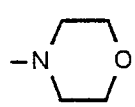 or 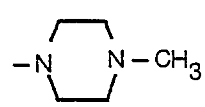

$R_5$ represents methyl and

$R_6$ represents phenyl or phenyl mono-substituted by fluorine, chlorine, $C_{1-4}$alkyl or $C_{1-4}$alkoxy.

3. A compound as claimed in Claim 1 or 2 wherein $R_1$ represents hydrogen, fluorine, methyl or methoxy.

4. A compound as claimed in claim 3 wherein $R_1$ represents hydrogen.

5. A compound as claimed in any one of Claims 1 to 4 wherein $R_3$ and $R_4$ represent methyl or together with the adjacent nitrogen atom

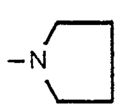 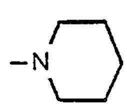 or 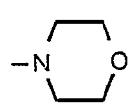 .

6. A compound as claimed in any one of Claims 1 to 5 wherein
a) $R_5$ and $R_6$ represent independently hydrogen or $C_{1-4}$alkyl,
b) $R_5$ represents methyl and $R_6$ represents phenyl,
c) $R_5$ represents methyl and $R_6$ represents $C_{1-4}$alkyl or
d) $R_5$ represent $C_{1-4}$alkyl and $R_6$ represents ethyl.

7. A compound as claimed in claim 6 wherein
a) $R_5$ and $R_6$ represent independently hydrogen or $C_{1-2}$alkyl,
b) $R_5$ represents methyl and $R_6$ represents phenyl,
c) $R_5$ represents methyl and $R_6$ represents ethyl or
d) $R_5$ represents $C_{1-4}$alkyl and $R_6$ represents ethyl.

8. $\alpha$-(Dimethylaminomethyl)-2-(3-ethyl-5-methyl-4-isoxazolyl)-1H-indole-3-methanol.

9. A compound as claimed in any one of Claims 1 to 8 in free base form or in the form of an acid addition or alkali-metal salt thereof.

10. A pharmaceutical composition comprising a compound as claimed in any one of Claims 1 to 8 in free base form or in the form of a pharmaceutically acceptable acid addition or alkali metal salt thereof in admixture with a pharmaceutically acceptable diluent or carrier.

11. A compound as claimed in any one of Claims 1 to 8 in free base form or in the form of a pharmaceutically acceptable acid addition or alkali metal salt thereof for use as a pharmaceutical.

12. A compound as claimed in any one of claims 1 to 8 in free base form or in the form of a pharmaceutically acceptable acid addition or alkali metal salt thereof for use as a hypoglycemic agent and/or an agent for inhibiting or impeding post-prandial hyperglycemia.

13. A process for the production of compounds of formula I or acid addition or alkali metal salts thereof, which comprises reducing the corresponding compound of formula II

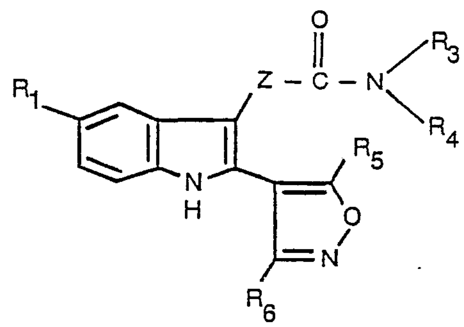

II

wherein
$R_1$, $R_3$, $R_4$, $R_5$ and $R_6$ are as defined in Claim 1,
Z signifies —CO— or —$CH_2$—.
and if desired converting a compound thus obtained into an acid addition or alkali metal salt thereof or vice versa.

**Claims for the Contracting State: AT**

1. A process for the production of compounds of formula I

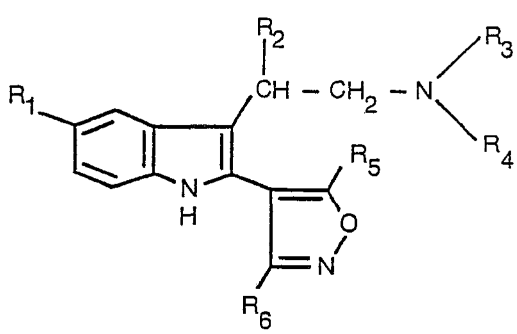

I

wherein
$R_1$ represents hydrogen, fluorine, chlorine, $C_{1-4}$alkyl or $C_{1-4}$alkoxy,
$R_2$ represents hydrogen or hydroxyl,
$R_3$ and $R_4$ represent, independently, $C_{1-4}$alkyl or together with the adjacent nitrogen atom

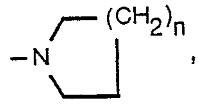 , 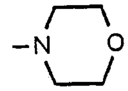 or 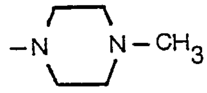

wherein

n is 1, 2 or 3,

$R_5$ represents hydrogen or $C_{1-4}$alkyl and

$R_6$ represents hydrogen, $C_{1-4}$alkyl, phenyl or phenyl mono-substituted by fluorine, chlorine, $C_{1-4}$alkyl or $C_{1-4}$alkoxy, or acid addition or alkali metal salts thereof, which comprises reducing the corresponding compound of formula II

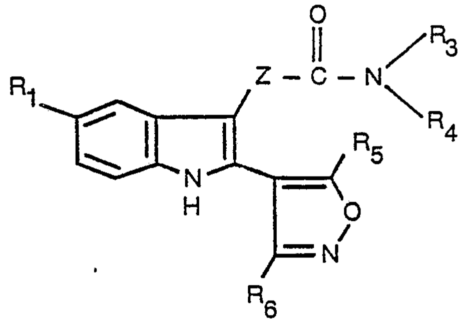

II

wherein

$R_1$, $R_3$, $R_4$, $R_5$ and $R_6$ are as defined above and

Z signifies —CO— or —$CH_2$—,

and if desired converting a compound thus obtained into an acid addition or alkali metal salt thereof or vice versa.

2. A process as claimed in Claim 1 wherein

a) $R_3$ and $R_4$ represent independently $C_{1-4}$alkyl or together with the adjacent nitrogen atom

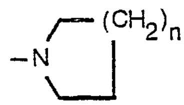 or 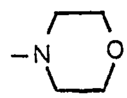 ,

and

$R_5$ and $R_6$ represent independently hydrogen or $C_{1-4}$alkyl,

b) $R_3$ and $R_4$ represent independently $C_{1-4}$alkyl or together with the adjacent nitrogen atom

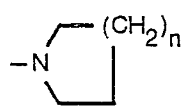 or 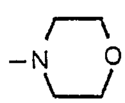 ,

and

$R_2$ represents hydroxyl or

c) $R_2$ represent hydrogen, $R_3$ and $R_4$ independently represent $C_{1-4}$alkyl or together with the adjacent nitrogen atom

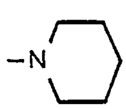 , 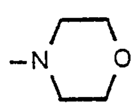 or 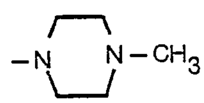

$R_5$ represents methyl and

$R_6$ represents phenyl or phenyl mono-substituted by fluorine, chlorine, $C_{1-4}$alkyl or $C_{1-4}$alkoxy.

3. A process as claimed in Claim 1 or 2 wherein $R_1$ represents hydrogen, fluorine, methyl or methoxy.

4. A process as claimed in Claim 3 wherein $R_1$ represents hydrogen.

5. A process as claimed in any one of Claims 1 to 4 wherein $R_3$ and $R_4$ represent methyl or together with the adjacent nitrogen atom

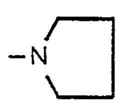 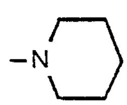 or 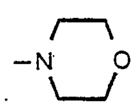 .

6. A process as claimed in any one of Claims 1 to 5 wherein

a) $R_5$ and $R_6$ represent independently hydrogen or $C_{1-4}$alkyl

b) $R_5$ represents methyl and $R_6$ represents phenyl,

c) $R_5$ represents methyl and $R_6$ represents $C_{1-4}$alkyl, or

d) $R_5$ represent $C_{1-4}$alkyl and $R_6$ represents ethyl.

7. A process as claimed in Claim 6 wherein

a) $R_5$ and $R_6$ represent independently hydrogen or $C_{1-2}$alkyl,

b) $R_5$ represents methyl and $R_6$ represents phenyl

c) $R_5$ represents methyl and $R_6$ represents ethyl or

d) $R_5$ represents $C_{1-4}$methyl and $R_6$ represents ethyl.

8. A process according to Claim 1 wherein the compound prepared is $\alpha$-(Dimethylaminomethyl)-2-(3-ethyl-5-methyl-4-isoxazolyl)-1H-indole-3-methanol or an acid addition or alkali metal salt thereof.

9. A pharmaceutical composition comprising a compound prepared according to any one of Claims 1 to 8 in free base form or in the form of a pharmaceutically acceptable acid addition or alkali metal salt thereof in admixture with a pharmaceutically acceptable diluent or carrier.

10. A compound prepared according to any one of Claims 1 to 8 in free base form or in the form of a pharmaceutically acceptable acid addition or alkali metal salt thereof for use as a pharmaceutical.

11. A compound prepared according to any one of Claims 1 to 8 in free base form or in the form of a pharmaceutically acceptable acid addition or alkali metal salt thereof for use as a hypoglycemic agent and/or an agent for inhibiting or impeding postprandial hyperglycemia.

12. A process for preparing a pharmaceutical composition which comprises admixing a compound of formula I

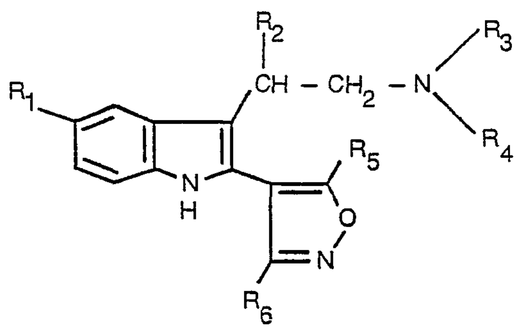

I

wherein

$R_1$ represents hydrogen, fluorine, chlorine, $C_{1-4}$alkyl or $C_{1-4}$alkoxy,

$R_2$ represents hydrogen or hydroxyl,

$R_3$ and $R_4$ represent, independently, $C_{1-4}$alkyl or together with the adjacent nitrogen atom

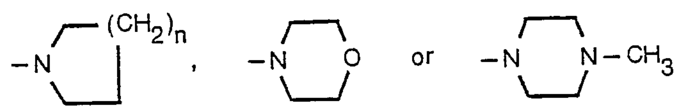

, or

wherein

n is 1, 2 or 3,

$R_5$ represents hydrogen or $C_{1-4}$alkyl and

$R_6$ represents hydrogen, $C_{1-4}$alkyl, phenyl or phenyl mono-substituted by fluorine, chlorine, $C_{1-4}$alkyl or $C_{1-4}$alkoxy,

in free base form or in the form of a pharmaceutically acceptable acid addition or alkali metal salt thereof with a pharmaceutically acceptable diluent or carrier.

**Revendications pour les Etats contractants: BE CH DE FR GB IT LI LU NL SE**

1. Composés de formule I

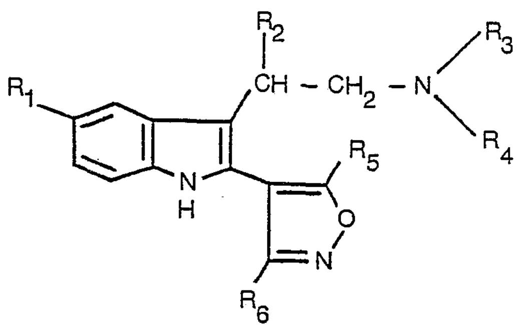

I

dans laquelle

$R_1$ représente l'hydrogène, le fluor, le chlore, $C_1$—$C_4$-alkyle ou $C_1$—$C_4$-alcoxy,

$R_2$ représente l'hydrogène ou hydroxyle,

$R_3$ et $R_4$ représentent, indépendamment l'un de l'autre, $C_1$—$C_4$-alkyle ou ensemble avec l'atome d'azote adjacent

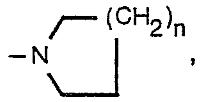 , 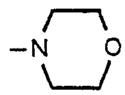 ou 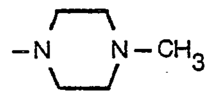

dans lequel

n signifie 1, 2 ou 3,

$R_5$ représente l'hydrogène ou $C_1$—$C_4$-alkyle et

$R_6$ représente l'hydrogène, $C_1$—$C_4$-alkyle, phényle ou phényle mono-substitué par le fluor, le chlore, $C_1$—$C_4$-alkyle ou $C_1$—$C_4$-alcoxy.

2. Un composé tel que revendiqué à la revendication 1, dans lequel

a) $R_3$ et $R_4$ représentent indépendamment l'un de l'autre $C_1$—$C_4$-alkyle ou ensemble avec l'atome d'azote adjacent

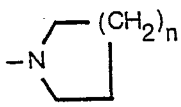 ou 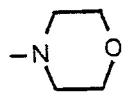 ,

et

$R_5$ et $R_6$ représentent indépendamment l'un de l'autre l'hydrogène ou $C_1$—$C_4$-alkyle,

b) $R_3$ et $R_4$ représentent indépendamment l'un de l'autre $C_1$—$C_4$-alkyle ou ensemble avec l'atome d'azote adjacent

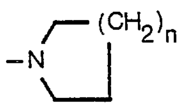 ou 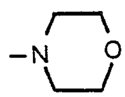 ,

et

$R_2$ représente hydroxyle ou

c) $R_2$ représente l'hydrogène, $R_3$ et $R_4$ représentent indépendamment l'un de l'autre $C_1$—$C_4$-alkyle ou ensemble avec l'atome d'azote adjacent

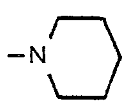 , 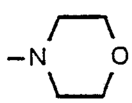 ou 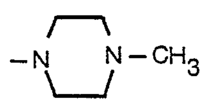

$R_5$ représente méthyle et

$R_6$ représente phényle ou phényle monosubstitué par le fluor, le chlore, $C_1$—$C_4$-alkyle ou $C_1$—$C_4$-alcoxy.

3. Un composé tel que revendiqué à la revendication 1 ou 2, dans lequel $R_1$ représente l'hydrogène, le fluor, méthyle ou méthoxy.

4. Un composé tel que revendiqué à la revendication 3, dans lequel $R_1$ représente l'hydrogène.

5. Un composé tel que revendiqué à l'une quelconque des revendications 1 à 4, dans lequel $R_3$ et $R_4$ représentent méthyle ou ensemble avec l'atome d'azote adjacent

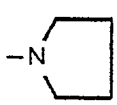 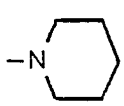 ou 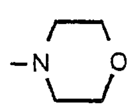 .

6. Un composé tel que revendiqué à l'une quelconque des revendications 1 à 5, dans lequel

a) $R_5$ et $R_6$ représentent indépendamment l'un de l'autre l'hydrogène ou $C_1$—$C_4$-alkyle,

b) $R_5$ représente méthyle et $R_6$ représente phényle,

c) $R_5$ représente méthyle et $R_6$ représente $C_1$—$C_4$-alkyle ou

d) $R_5$ représente $C_1$—$C_4$-alkyle et $R_6$ représente éthyle.

7. Un composé tel que revendiqué à la revendication 6, dans lequel

a) $R_5$ et $R_6$ représentent indépendamment l'un de l'autre l'hydrogène ou $C_1$—$C_2$-alkyle

b) $R_5$ représente méthyle et $R_6$ représente phényle

c) $R_5$ représente méthyle et $R_6$ représente éthyle ou

d) $R_5$ représente $C_1$—$C_4$-alkyle et $R_6$ représente éthyle.

8. $\alpha$-(diméthylaminométhyl)-2-(3-éthyl-5-méthyl-4-isoxazolyl)-1H-indole-3-méthanol.

9. Un composé tel que revendiqué à l'une quelconque des revendications 1 à 8, sous forme de base libre ou sous la forme d'un sel d'addition d'acide ou de métal alcalin de ce composé.

10. Une composition pharmaceutique comprenant un composé tel que revendiqué à l'une quelconque des revendications 1 à 8 sous forme de base libre ou sous la forme d'un sel d'addition d'acide ou de métal alcalin pharmaceutiquement acceptable de ce composé, en mélange avec un diluant ou véhicule pharmaceutiquement acceptable.

11. Un composé tel que revendiqué à l'une quelconque des revendications 1 à 8 sous forme de base libre ou sous la forme d'un sel d'addition d'acide ou de métal alcalin pharmaceutiquement acceptable de ce composé, pour l'utilisation comme médicament.

12. Un composé tel que revendiqué à l'une quelconque des revendications 1 à 8 sous forme de base libre ou sous la forme d'un sel d'addition d'acide ou de métal alcalin pharmaceutiquement acceptable de ce composé, pour l'utilisation comme agent hypoglycémiant et/ou comme agent pour inhiber ou empêcher l'hyperglycémie post-prandiale.

13. Un procédé de préparation des composés de formule I ou de leurs sels d'addition d'acides ou de métaux alcalins, qui comprend la réduction du composé correspondant de formule II

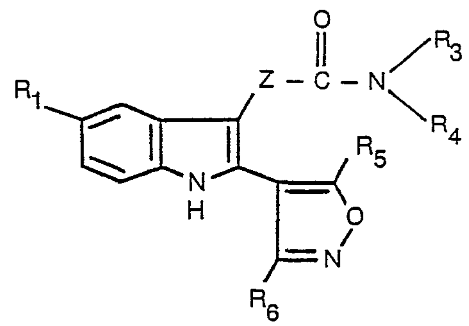

II

dans laquelle

$R_1$, $R_3$, $R_4$, $R_5$ et $R_6$ sont tels que définis à la revendication 1,

Z signifie —CO— ou —$CH_2$—

et, si désiré, la transformation d'un composé ainsi obtenu en un sel d'addition d'acide ou de métal alcalin de ce composé, ou vice versa.

**Revendications pour l'Etat contractant: AT**

1. Un procédé de préparation des composés de formule I

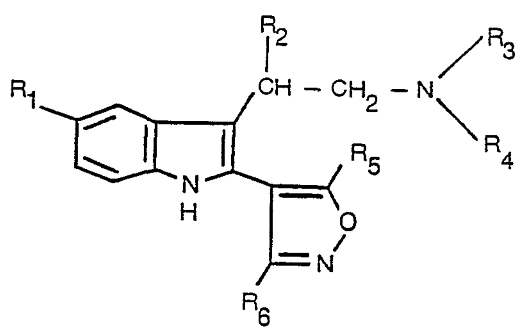

I

dans laquelle

$R_1$ représente l'hydrogène, le fluor, le chlore, $C_1$—$C_4$-alkyle ou $C_1$—$C_4$-alcoxy,

$R_2$ représente l'hydrogène ou hydroxyle,

$R_3$ et $R_4$ représentent, indépendamment l'un de l'autre, $C_1$—$C_4$-alkyle ou ensemble avec l'atome d'azote adjacent

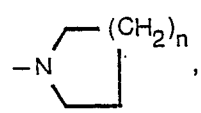 , 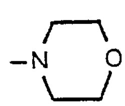 ou 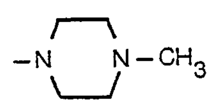

dans lequel

n signifie 1, 2 ou 3,

$R_5$ représente l'hydrogène ou $C_1$—$C_4$-alkyle et

$R_6$ représente l'hydrogène, $C_1$—$C_4$-alkyle, phényle ou phényle monosubstitué par le fluor, le chlore, $C_1$—$C_4$-alkyl ou $C_1$—$C_4$-alcoxy ou de leurs sels d'addition d'acides ou de métaux alcalins, qui comprend la réduction du composé correspondant de formule II

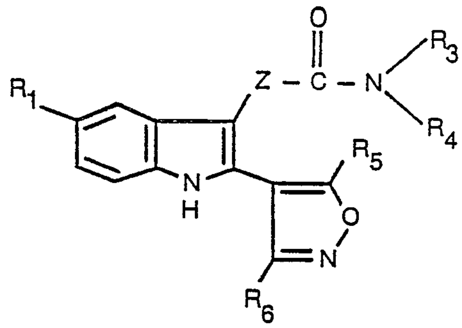 II

dans laquelle

$R_1$, $R_3$, $R_4$, $R_5$ et $R_6$ sont tels que définis plus haut et

Z signifie —CO— ou —$CH_2$—,

et, si désiré, la transformation d'un composé ainsi obtenu en un sel d'addition d'acide ou de métal alcalin de ce composé ou vice versa.

2. Un procédé tel que revendiqué à la revendication 1, dans lequel

a) $R_3$ et $R_4$ représentent indépendamment l'un de l'autre $C_1$—$C_4$-alkyle ou ensemble avec l'atome d'azote adjacent

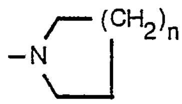 ou 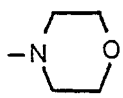 ,

et

$R_5$ et $R_6$ représentent indépendamment l'un de l'autre l'hydrogène ou $C_1$—$C_4$-alkyle,

b) $R_3$ et $R_4$ représentent indépendamment l'un de l'autre $C_1$—$C_4$-alkyle ou ensemble avec l'atome d'azote adjacent

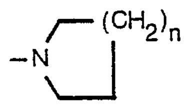 ou 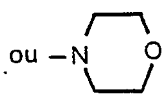 ,

et

$R_2$ représente hydroxyle ou

c) $R_2$ représente l'hydrogène, $R_3$ et $R_4$ représentent indépendamment l'un de l'autre $C_1$—$C_4$-alkyle ou ensemble avec l'atome d'azote adjacent

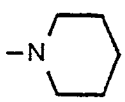 , 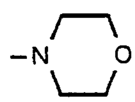 ou 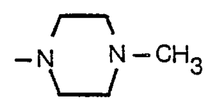

$R_5$ représente méthyle et

$R_6$ représente phényle ou phényle monosubstitué par le fluor, le chlore, $C_1$—$C_4$-alkyle ou $C_1$—$C_4$-alcoxy.

3. Un procédé tel que revendiqué à la revendication 1 ou 2, dans lequel $R_1$ représente l'hydrogène, le fluor, méthyle ou méthoxy.

4. Un procédé tel que revendiqué à la revendication 3, dans lequel $R_1$ représente l'hydrogène.

5. Un procédé tel que revendiqué à l'une quelconque des revendications 1 à 4, dans lequel $R_3$ et $R_4$ représentent méthyle ou ensemble avec l'atome d'azote adjacent

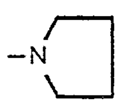 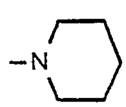 ou 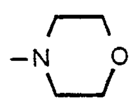 .

6. Un procédé tel que revendiqué à l'une quelconque des revendications 1 à 5, dans lequel

a) $R_5$ et $R_6$ représentent indépendamment l'un de l'autre l'hydrogène ou $C_1$—$C_4$-alkyle

b) $R_5$ représente méthyle et $R_6$ représente phényle,

c) $R_5$ représente méthyle et $R_6$ représente $C_1$—$C_4$-alkyle, ou

d) $R_5$ représente $C_1$—$C_4$-alkyle et $R_6$ représente éthyle.

7. Un procédé tel que revendiqué à la revendication 6, dans lequel

a) $R_5$ et $R_6$ représentent indépendamment l'un de l'autre l'hydrogène ou $C_1$—$C_2$-alkyle,

b) $R_5$ représente méthyle et $R_6$ représente phényle

c) $R_5$ représente méthyle et $R_6$ représente éthyle ou

d) $R_5$ représente $C_1$—$C_4$-méthyle et $R_6$ représente éthyle.

8. Un procédé selon la revendication 1, dans lequel le composé préparé est le $\alpha$-(diméthylaminométhyl)-2-(3-éthyl-5-méthyl-4-isoxazolyl)-1H-indole-3-méthanol ou un sel d'addition d'acide ou de métal alcalin de ce composé.

9. Une composition pharmaceutique comprenant un composé préparé selon l'une quelconque des revendications 1 à 8 sous forme de base libre ou sous la forme d'un sel d'addition d'acide ou de métal alcalin pharmaceutiquement acceptable de ce composé, en mélange avec un diluant ou véhicule pharmaceutiquement acceptable.

10. Un composé préparé selon l'une quelconque des revendications 1 à 8 sous forme de base libre ou sous la forme d'un sel d'addition d'acide ou de métal alcalin pharmaceutiquement acceptable de ce composé, pour l'utilisation comme médicament.

11. Un composé préparé selon l'une quelconque des revendications 1 à 8 sous forme de base libre ou sous la forme d'un sel d'addition d'acide ou de métal alcalin pharmaceutiquement acceptable de ce composé, pour l'utilisation comme agent hypoglycémiant et/ou comme agent pour inhiber ou empêcher l'hyperglycémie post-prandiale.

12. Un procédé de préparation d'une composition pharmaceutique qui comprend l'admixtion d'un composé de formule I

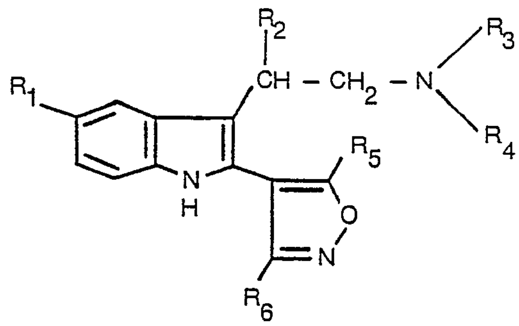

I

dans laquelle

$R_1$ représente l'hydrogène, le fluor, le chlore, $C_1$—$C_4$-alkyle ou $C_1$—$C_4$-alcoxy,

$R_2$ représente l'hydrogène ou hydroxyle,

$R_3$ et $R_4$ représentent, indépendamment l'un de l'autre, $C_1$—$C_4$-alkyle ou ensemble avec l'atome d'azote adjacent

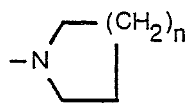

, 

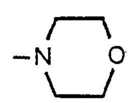

ou

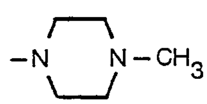

dans lequel

n signifie 1, 2 ou 3,

$R_5$ représente l'hydrogène ou $C_1$—$C_4$-alkyle et

$R_6$ représente l'hydrogène, $C_1$—$C_4$-alkyle, phényle ou phényle monosubstitué par le fluor, le chlore, $C_1$—$C_4$-alkyle ou $C_1$—$C_4$-alcoxy,

sous forme de base libre ou sous la forme d'un sel d'addition d'acide ou de métal alcalin pharmaceutiquement acceptable de ce composé, avec un diluant ou véhicle pharmaceutiquement acceptable.

**Patentansprüche für die Vertragsstaaten: BE CH DE FR GB IT LI LU NL SE**

1. Verbindungen der Formel I

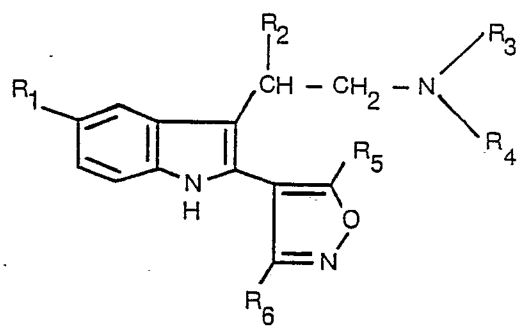

I

worin

$R_1$ Wasserstoff, Fluor, Chlor, $C_{1-4}$Alkyl oder $C_{1-4}$Alkoxy,

$R_2$ Wasserstoff oder Hydroxyl,

$R_3$ und $R_4$, unabhängig, $C_{1-4}$Alkyl oder zusammen mit dem benachbarten Stickstoffatom

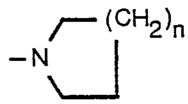 , 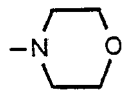 oder 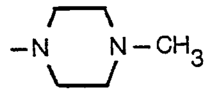

worin

n 1, 2 oder 3 ist,

$R_5$ Wasserstoff oder $C_{1-4}$Alkyl und

$R_6$ Wasserstoff, $C_{1-4}$Alkyl, Phenyl oder Phenyl mono-substituiert durch Fluor, Chlor, $C_{1-4}$Alkyl oder $C_{1-4}$Alkoxy, darstellen.

2. Eine Verbindung wie in Anspruch 1 beansprucht, worin

a) $R_3$ und $R_4$, unabhängig, $C_{1-4}$Alkyl oder zusammen mit dem benachbarten Stickstoffatom

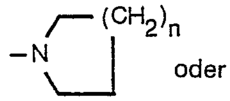 oder 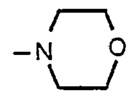 ,

$R_5$ und $R_6$, unabhängig Wasserstoff oder $C_{1-4}$Alkyl, darstellen.

b) $R_3$ und $R_4$, unabhängig, $C_{1-4}$Alkyl oder zusammen mit dem benachbarten Stickstoffatom

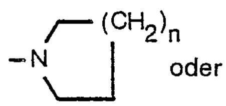 oder 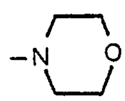 und

$R_2$ Hydroxyl, darstellen,

c) $R_2$ Wasserstoff, $R_3$ und $R_4$, unabhängig, $C_{1-4}$Alkyl oder zusammen mit dem benachbarten Stickstoffatom

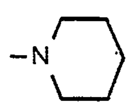 , 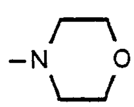 oder 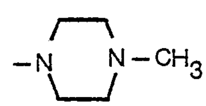

$R_5$ Methyl und

$R_6$ Phenyl oder Phenyl mono-substituiert durch Fluor, Chlor, $C_{1-4}$Alkyl oder $C_{1-4}$Alkoxy, darstellen.

3. Eine Verbindung wie im Anspruch 1 oder 2 beansprucht, worin $R_1$ Wasserstoff, Fluor, Methyl oder Methoxy darstellt.

4. Eine Verbindung wie im Anspruch 3 beansprucht, worin $R_1$ Wasserstoff darstellt.

5. Eine Verbindung wie in einem der Ansprüche 1 bis 4 beansprucht, worin $R_3$ und $R_4$ Methyl oder zusammen mit dem benachbarten Stickstoffatom

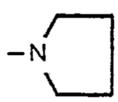 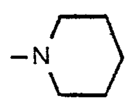 oder 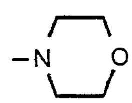 ,

darstellen.

6. Eine Verbindung wie in einem der Ansprüche 1 bis 5 beansprucht, worin

a) $R_5$ und $R_6$, unabhängig, Wasserstoff oder $C_{1-4}$Alkyl,

b) $R_5$ Methyl und $R_6$ Phenyl,

c) $R_5$ Methyl und $R_6$ $C_{1-4}$Alkyl oder

d) $R_5$ $C_{1-4}$Alkyl und $R_6$ Ethyl darstellen.

7. Eine Verbindung wie im Anspruch 6 beansprucht, worin

a) $R_5$ und $R_6$, unabhängig, Wasserstoff oder $C_{1-2}$Alkyl,

b) $R_5$ Methyl und $R_6$ Phenyl,

c) $R_5$ Methyl und $R_6$ Ethyl oder

d) $R_5$ $C_{1-4}$Alkyl und $R_6$ Ethyl darstellen.

8. $\alpha$-(Dimethylaminomethyl)-2-(3-ethyl-5-methyl-4-isoxazolyl)-1H-indole-3-methanol.

9. Eine Verbindung wie in einem der Ansprüche 1 bis 8 beansprucht in Form der freien Base oder in Form eines Säureadditions- oder Alkalimetallsalzes, derselben.

10. Eine pharmazeutische Zusammensetzung enthaltend eine Verbindung wie in einem der Ansprüche 1 bis 8 beansprucht in Form der freien Base oder in Form eines pharmazeutisch annehmbaren Säureadditions- oder Alkalimetallsalzes derselben unter Beimischung von einem pharmazeutisch annehmbaren Verdünner oder Träger.

11. Eine Verbindung wie in einem der Ansprüche 1 bis 8 beansprucht in Form der freien Base oder in Form eines pharmazeutisch annehmbaren Säureadditions- oder Alkalimetallsalzes derselben zur Verwendung als Pharmazeutikum.

12. Eine Verbindung wie in einem der Ansprüche 1 bis 8 beansprucht in Form der freien Base oder in Form eines pharmazeutisch annehmbaren Säureadditions- oder Alkalimetallsalzes derselben zur Verwendung als ein hypoglkämisches Mittel und/oder als Mittel zur Verhinderung oder Hemmung von Hyperglkämie nach dem Essen.

13. Verfahren zur Herstellung von Verbindungen der Formel I oder Säureadditions- oder Alkalimetallsalzen derselben bestehend aus Reduzierung der entsprechenden Verbindung der Formel II

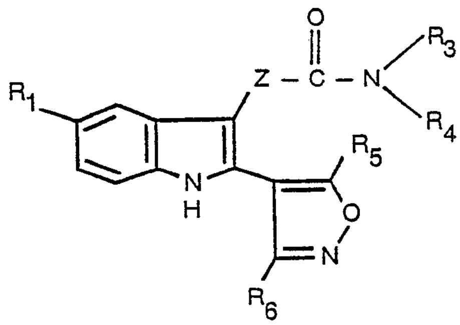

II

worin

$R_1$, $R_3$, $R_4$, $R_5$ und $R_6$ wie im Anspruch 1 definiert sind und

Z—CO— oder $CH_2$ bedeutet,

und falls erwünscht Umwandlung einer so erhaltenen Verbindung in ein Säureadditions- oder Alkalimetallsalz derselben oder umgekehrt.

**Patentansprüche für den Vertragsstaat AT**

1. Verfahren zur Herstellung von Verbindungen der Formel I

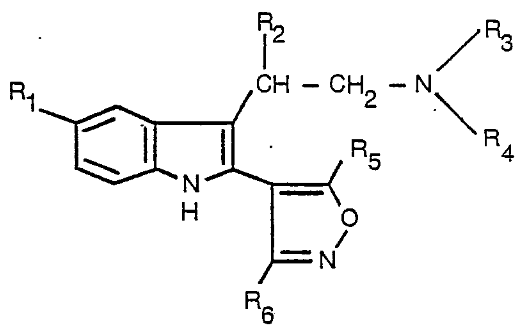

I

worin

$R_1$ Wasserstoff, Fluor, Chlor, $C_{1-4}$Alkyl oder $C_{1-4}$Alkoxy,

$R_2$ Wasserstoff oder Hydroxyl,

$R_3$ und $R_4$, unabhängig, $C_{1-4}$Alkyl oder zusammen mit dem benachbarten Stickstoffatom

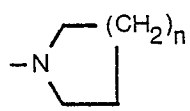

,

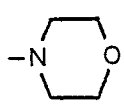

oder

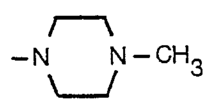

worin

n 1, 2 oder 3 ist,

$R_5$ Wasserstoff oder $C_{1-4}$Alkyl und

$R_6$ Wasserstoff, $C_{1-4}$Alkyl, Phenyl oder Phenyl mono-substituiert durch Fluor, Chlor, $C_{1-4}$Alkyl oder $C_{1-4}$Alkoxy, darstellen, oder Säureadditions- oder Alkalimetallsalzen derselben, bestehend aus Reduzierung der entsprechenden Verbindung der Formel II

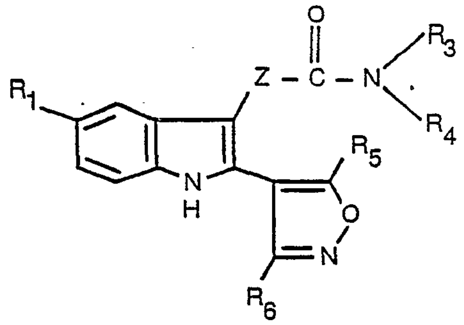

II

worin

$R_1$, $R_3$, $R_4$, $R_5$ und $R_6$ wie im Anspruch 1 definiert sind und

Z—CO— oder $CH_2$ bedeutet,

und falls erwünscht Umwandlung einer so erhaltenen Verbindung in ein Säureadditions- oder Alkalimetallsalz derselben oder umgekehrt.

2. Ein Verfahren wie im Anspruch 1 beansprucht, worin

a) $R_3$ und $R_4$, unabhängig, $C_{1-4}$Alkyl oder zusammen mit dem benachbarten Stickstoffatom

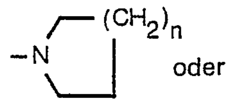 oder 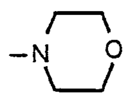 ,

$R_5$ und $R_6$, unabhängig Wasserstoff oder $C_{1-4}$Alkyl, darstellen,

b) $R_3$ und $R_4$, unabhängig, $C_{1-4}$Alkyl oder zusammen mit dem benachbarten Stickstoffatom

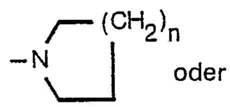 oder 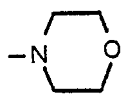 ,

und

$R_2$ Hydroxyl, darstellen,

c) $R_2$ Wasserstoff, $R_3$ und $R_4$, unabhängig, $C_{1-4}$Alkyl oder zusammen mit dem benachbarten Stickstoffatom

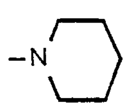 , 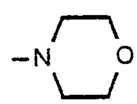 oder 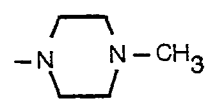

$R_5$ Methyl und

$R_6$ Phenyl oder Phenyl mono-substitutiert durch Fluor, Chlor, $C_{1-4}$Alkyl oder $C_{1-4}$Alkoxy, darstellen.

3. Ein Verfahren wie im Anspruch 1 oder 2 beansprucht, worin $R_1$ Wasserstoff, Fluor, Methyl oder Methoxy darstellt.

4. Eine Verbindung wie im Anspruch 3 beansprucht, worin $R_1$ Wasserstoff darstellt.

5. Ein Verfahren wie in einem der Ansprüche 1 bis 4 beansprucht, worin $R_3$ und $R_4$ Methyl oder zusammen mit dem benachbarten Stickstoffatom

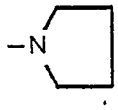 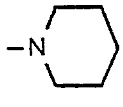 oder 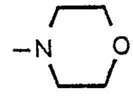 ,

darstellen.

6. Ein Verfahren wie in einem der Ansprüche 1 bis 5 beansprucht, worin

a) $R_5$ und $R_6$, unabhängig, Wasserstoff oder $C_{1-4}$Alkyl,

b) $R_5$ Methy und $R_6$ Phenyl,

c) $R_5$ Methyl und $R_6$ $C_{1-4}$Alkyl oder

d) $R_5$ $C_{1-4}$Alkyl und $R_6$ Ethyl darstellen.

7. Ein Verfahren wie im Anspruch 6 beansprucht, worin

a) $R_5$ und $R_6$, unabhängig, Wasserstoff oder $C_{1-2}$Alkyl,

b) $R_5$ Methyl und $R_6$ Phenyl,

c) $R_5$ Methyl und $R_6$ Ethyl oder

d) $R_5$ $C_{1-4}$Alkyl und $R_6$ Ethyl darstellen.

8. Ein Verfahren nach Anspruch 1, worin die hergestellte Verbindung $\alpha$-(Dimethylaminomethyl)-2-(3-ethyl-5-methyl-4-isoxazolyl)-1H-indole-3-methanol oder ein Säureadditions- oder Alkalimetallsalz derselben ist.

9. Eine pharmazeutische Zusammensetzung enthaltend eine nach einem der Ansprüche 1 bis 8 hergestellte Verbindung in Form der freien Base oder in Form eines pharmazeutisch annehmbaren Säureadditions- oder Alkalimetallsalzes derselben unter Beimischung von einem pharmazeutisch annehmbaren Verdünner oder Träger.

10. Eine nach einem der Ansprüche 1 bis 8 hergestellte Verbindung in Form der freien Base oder in Form eines pharmazeutisch annehmbaren Säureadditions- oder Alkalimetallsalzes derselben zur Verwendung als Pharmazeutikum.

11. Eine nach einem der Ansprüche 1 bis 8 hergestellte Verbindung in Form der freien Base oder in Form eines pharmazeutisch annehmbaren Säureadditions- oder Alkalimetallsalzes derselben zur Verwendung als ein hypoglykämisches Mittel und/oder als Mittel zur Verhinderung oder Hemmung von Hyperglykämie nach dem Essen.

12. Verfahren zur Herstellung einer pharmazeutischen Zusammensetzung bestehend aus Beimischung einer Verbindung der Formel I

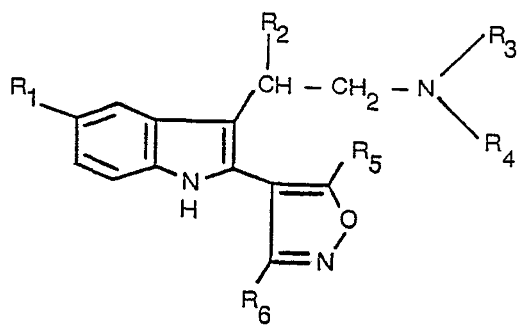

I

worin

$R_1$ Wasserstoff, Fluor, Chlor, $C_{1-4}$Alkyl oder $C_{1-4}$Alkoxy,

$R_2$ Wasserstoff oder Hydroxyl,

$R_3$ und $R_4$, unabhängig, $C_{1-4}$Alkyl oder zusammen mit dem benachbarten Stickstoffatom

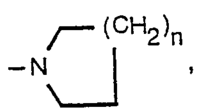 , 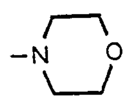 oder 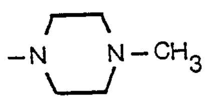

worin

n 1, 2 oder 3 ist,

$R_5$ Wasserstoff oder $C_{1-4}$Alkyl und

$R_6$ Wasserstoff, $C_{1-4}$Alkyl, Phenyl oder Phenyl mono-substituiert durch Fluor, Chlor, $C_{1-4}$Alkyl oder $C_{1-4}$Alkoxy,

darstellen, in Form der freien Base oder in Form eines Säureadditions- oder Alkalimetallsalzes mit einem pharmazeutisch annehmbaren Verdünner oder Träger.